(19) Europäisches Patentamt
European Patent Office
Office européen des brevets

(11) **EP 2 500 837 A1**

(12) **EUROPEAN PATENT APPLICATION**

(43) Date of publication:
**19.09.2012 Bulletin 2012/38**

(51) Int Cl.:
***G06F 19/24*** *(2011.01)*   *G06F 19/20* *(2011.01)*

(21) Application number: **11157794.6**

(22) Date of filing: **11.03.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Qlucore AB**
**223 70 Lund (SE)**

(72) Inventors:
• **Fontes, Magnus**
  **224 75, Lund (SE)**
• **Soneson, Charlotte**
  **226 43, Lund (SE)**

(74) Representative: **Ström & Gulliksson AB**
**Box 4188**
**203 13 Malmö (SE)**

(54) **Method for robust comparison of data**

(57) The present invention provides relatively robust comparison of data, such as but not limited to gene expression data, by providing a computer-implemented method, a computer program product and a computer readable medium, that analyzes data according to the appended patent claims. The invention provides stability with respect to re-sampling of data.

Fig. 1

**Description**

**Technical Field**

This invention pertains in general to the field of data processing. More particularly the invention relates to a computer-implemented method for analyzing multivariate data.

**Background**

In many modern research areas, huge data sets are being routinely produced. To interpret these data sets and yield useful results, researchers often have to compare the experimentally obtained data to previously extracted information, which is collected in large databases. The information in databases is often represented in the form of a list of objects, which is then compared to a list of data obtained from the experiment.

One area where high-throughput experiments generating vast amounts of data are ubiquitous is genetics, where microarray techniques are routinely used for studying e.g. genome-wide patterns of gene expression and copy number alterations. The output of univariate analysis of such high-throughput experiments is often a list comprising genes related to some phenotype of interest. In order to interpret the obtained list it is important to place it in a relevant biological context. An inherent problem with trying to interpret gene lists is that they are often highly unstable, both with regards to small changes in the underlying data set and with regards to changes in the method used to sort or rank them, i.e. the ranking method. This could be due to redundancy in the cell machinery, i.e. the existence of many genes having similar functions in the cell and thereby being exchangeable in a given experimental list. Current methods for list comparison does not account for this effect, i.e. the resulting lists are unstable, which can lead to biological conclusions which are highly variable and difficult to interpret. Methods according to prior art usually utilize external secondary information to draw statistical conclusions of data, such as utilizing groupings of genes to draw conclusions of genes. This makes them susceptible to multiple sources of error.

Other specific fields of technology generating vast amounts of data, such as meteorology, astronomy etc. each has specific limitations which contribute to unstable lists.

**Summary of the Invention**

An objective of the present invention is to provide relatively robust comparison of data, such as but not limited to gene expression data, by providing a computer-implemented method and a computer-readable medium, that analyze data according to the appended patent claims.

A general solution according to embodiments of the invention is to use information extracted from the experimental data to obtain a more stable list, which is represented in a form allowing straightforward comparisons to other lists of predetermined data, both in ordered and unordered form.

According to a first aspect, a computer-implemented method for robust comparison of data is provided, said method comprising a plurality $i$ of samples of each of a plurality of measurement variables g, based on a ranking method for sorting said data. The computer-implemented method comprising a first step of sorting the data into an ordered list $\ell$ according to the ranking provided by the ranking method; a second step of computing an exchangeability score of subsets of variables of the ordered data, resulting in an exchangeability matrix $V_\ell$; a third step of expanding the ordered lists $\ell$ of the data; and a fourth step of comparing the expanded experimental list vector $l_\ell$ with another list vector comprising a plurality of measurement variables g.

In an embodiment of the computer-implemented method according to the first aspect, the third step of expanding comprises the steps of computing a diagonal position matrix $A_\ell$, by defining diagonal elements as:

$$(A_i)_{ii} = u(r_i)$$

where $r_i$ is the ranking statistic of variable $i$ used in sorting the data into an ordered list $\ell$, and $u : \mathbb{R} \to \mathbb{R}$ is a monotone function; computing a diagonal global weight matrix $W_\ell$, by weighting the data; and computing an expanded experimental list vector $l_\ell$ according to a function:

$$f(A_\ell, V_\ell, W_\ell) = l_\ell.$$

In an embodiment of the computer-implemented method according to the first aspect, the computing of an expanded experimental list vector $l_\ell$ is done according to the formula:

$$l_\ell = ((l_\ell)_1, \ldots, (l_\ell)_M)$$

by letting $(l_\ell)_i = h((G_\ell)_i)$, where $G_\ell = A_\ell V_\ell W_\ell$; and $(G_\ell)_i$ is the $i$:th column of $G_\ell$ and $h$: $\mathbb{R}^M \to \mathbb{R}$.

In an embodiment of the computer-implemented method according to the first aspect, the subsets of variables are pairs of variables.

The pairs of variables may be pairs of variables with an exchangeability score exceeding a predefined threshold value.

In an embodiment of the computer-implemented method according to the first aspect, the ranking method is signal-to-noise ratio (SNR), fold change of average expression value , t-test, ANOVA, and non-parametric tests.

In an embodiment of the computer-implemented method according to the first aspect, the second step of computing an exchangeability score of each pair of variables is total exchangeability variation score, PS, mean exchangeability score, ES(mean), maximal exchangeability score, ES(max), one-sided mean exchangeability score, oES (mean), one-sided maximal exchangeability score, oES(max), or normalized variants thereof.

In an embodiment of the computer-implemented method according to the first aspect, computing the global weight matrix is according to the formula:

$$(W_\ell)_{ii} = \log\left(\frac{N+1}{N_i+1}\right)$$

where $N$ is the number of lists used as reference data and $N_i$ is the number of reference lists comprising $g_i$.

In an embodiment of the computer-implemented method according to the first aspect, $h$ is $h_1(x) = \Sigma^M_{i=1}x_i$; $h_2(x) = \min_{1 \le i \le M}|x_i|$; or $h_3(x) = ||x||$ for some norm $||\cdot||$ on $\mathbb{R}^M$.

In an embodiment of the computer-implemented method according to the first aspect, the fourth step of comparing the expanded experimental list vector $l_\ell$, called $l_{\ell 1}$, with the other list vector comprising a plurality of measurement variables g, called $l_{\ell 2}$, is done by computing a similarity coefficients $s(\ell_1, \ell_2)$ according to the formula

$$s(\ell_1, \ell_2) = \frac{l_{\ell_1} \cdot l_{\ell_2}}{\|l_{\ell_1}\|\|l_{\ell_2}\|};$$

and a dissimilarity coefficient $d(\ell_1, \ell_2)$ according to the formula

$$d(\ell_1, \ell_2) = 1 - s(\ell_1, \ell_2).$$

In an embodiment of the computer-implemented method according to the first aspect, the measurement variables are of the same kind, such as gene expression data.

In another embodiment of the computer-implemented method according to the first aspect, the measurement variables are of different kinds.

According to a second aspect, a computer program product is provided, comprising computer program code means for executing the method according the first aspect, when said computer program code means are run by an electronic device having computer capabilities.

According to a third aspect, a computer readable medium is provided, having stored thereon a computer program product comprising computer program code means for executing the method according to the first aspect, when said computer program code means are run by an electronic device having computer capabilities.

According to a fourth aspect, a computer is provided, configured to perform the method according the first aspect.

According to a fifth aspect, a method for determining a relationship between a plurality of physical and/or biological parameters is provided, comprising obtaining a plurality $i$ of samples of each of a plurality of measurement variables g, and a ranking method; and analyzing the samples of measurements using the method according to the first aspect.

In an embodiment of the method for determining a relationship between a plurality of physical and/or biological parameters according to the fifth aspect, the physical parameters are gene expression data.

Embodiments of the present invention have the advantage over the prior art that it enables relatively robust conclusions to be drawn from the data, since they provide stability with respect to re-sampling of data. A technical advantage of this is for example to enable improved analysis of data, such as genetic data.

**Brief Description of the Drawings**

These and other aspects, features and advantages of which the invention is capable of will be apparent and elucidated from the following description of embodiments of the present invention, reference being made to the accompanying drawings, in which

Fig. 1 is a schematic overview of the method steps according to an embodiment;
Fig. 2 is a plot illustrating exchangeability according to an embodiment, for two pairs of random variables;
Fig. 3 is a result plot according to an embodiment of the invention;
Figs 4-5 are comparative result plots;
Fig. 6 is a table showing the result according to an embodiment;
Figs 7-8 are concordance plots showing the result according to an embodiment; and
Fig. 9 is a schematic illustration of an embodiment.

**Description of embodiments**

Several embodiments of the present invention will be described in more detail below with reference to the accompanying drawings in order for those skilled in the art to be able to carry out the invention. The invention may, however, be embodied in many different forms and should not be construed as limited to the embodiments set forth herein. Rather, these embodiments are provided so that this disclosure will be thorough and complete, and will fully convey the scope of the invention to those skilled in the art. The embodiments do not limit the invention, but the scope of the invention is only limited by the appended patent claims. Furthermore, the terminology used in the detailed description of the particular embodiments illustrated in the accompanying drawings is not intended to be limiting of the invention.

**Exchangeability of random variables**

Consider a probability triple $(\Omega, F, P)$ and let $X_1, \ldots, X_m$ denote random variables on $\Omega$, taking values in some space M. Given $X_1, \ldots, X_m$ we define the multivariate random variable

$$X_1 \times \ldots \times X_m : \Omega \to \underbrace{\mathbb{M} \times \ldots \times \mathbb{M}}_{m}$$

by $X_1 \times \ldots \times X_m (\omega) = (X_1(\omega),\ldots,X_m(\omega))$. To each random variable $X_1 \times \ldots \times X_m$ there is an associated measure $Pr_{X_1 \times \ldots \times X_m}$ defined by

$$Pr_{X_1 \times \ldots \times X_m}(A) = P(\{\omega \in \Omega;\ X_1 \times \ldots \times X_m(\omega) \in A\})$$

for $A \subseteq M \times \ldots \times M$.

The concept of exchangeability of random variables was made popular by de Finetti in the 1930's. Conventionally, a finite sequence $(X_1, \ldots, X_m)$ of random variables is called exchangeable if their joint distribution is invariant under permutation of $X_1, \ldots, X_m$, i.e. if

$$Pr_{X_1 \times \ldots \times X_m} = Pr_{X_{\pi(1)} \times \ldots \times X_{\pi(m)}}$$

for each $\pi \in S_m$ (the group of permutations of $\{1,\ldots,m\}$). This means that from a statistical point of view the order of the variables in the product is completely irrelevant. From this definition, it is clear that any sequence of independent and identically distributed (i.i.d.) random variables is exchangeable, but the reverse implication is false. The definition of exchangeability given above is rather strong, and we introduce a much weaker notion of exchangeability as follows:

*Definition 1.* The finite sequence of random variables $(X_1, ... , X_m)$ is weakly exchangeable if the null sets of $Pr_{X_{1 \times ... \times X_m}}$ are invariant under permutations, i.e.

$$Pr_{X_{\pi(1)} \times ... \times X_{\pi(m)}} \ll Pr_{X_{\tau(1)} \times ... \times X_{\tau(m)}}$$

for all $\pi, \tau \in S_m$. Here, $\mu \ll \nu$ denotes that the positive measure $\mu$ is absolutely continuous with respect to the positive measure $\nu$.

It is clear that a finite sequence of random variables $(X_1, ... , X_m)$ that is exchangeable is weakly exchangeable, but that the opposite implication is false in general.

**Measure of exchangeability**

In this section we will discuss some ways to quantify the degree of exchangeability for a sequence of random variables.

*Definition 2.* Given a finite sequence of random variables $(X_1, ..., X_m)$, the total exchangeability variation is given by

$$P^{Var}_{X_1 \times ... \times X_m} := \frac{1}{|S_m| - 1} \sum_{\pi \in S_m} \left| Pr_{X_{\pi(1)} \times ... \times X_{\pi(m)}} - \frac{1}{|S_m|} \sum_{\tau \in S_m} Pr_{X_{\tau(1)} \times ... \times X_{\tau(m)}} \right|.$$

Here, $| \mu |$ denotes the total variation of the (real-valued) measure $\mu$.

The total exchangeability variation is a positive measure and we note that $P^{Var}_{X_{1 \times ... \times X_m}} (\Omega) = 0$ if and only if the sequence $(X_1, ... , X_m)$ is exchangeable.

We now turn to a discrete probability space $(\Omega, F, P)$, where $\Omega$ is a finite set, $F = 2^\Omega$ is the $\sigma$-algebra consisting of all events and $P:F \to [0,1]$ is a probability measure.

We let $X_1, ... , X_m$ be random variables on $\Omega$ taking values in M $:= \{1, ..., M\}$. The support of the random variable $X_1 \times ... \times X_m$ is defined by

$$\text{supp } X_1 \times ... \times X_m := \left\{ (q_1, ..., q_m) \in \mathbb{M} \times ... \times \mathbb{M}; \ Pr_{X_1 \times ... \times X_m}(\{(q_1, ..., q_m)\}) > 0 \right\}$$

For finite sets of discrete random variables, Definition 1 implies that $(X_1, ... , X_m)$ is weakly exchangeable if

$$\text{supp}(X_1 \times ... \times X_m) = \text{supp}\left(X_{\pi(1)} \times ... \times X_{\pi(m)}\right)$$

for all Therefore, to quantify the degree of weak exchangeability for a set of discrete random variables we will compare the support of the joint distributions.

Let $\rho: (M x ... x M) x (M x ... x M) \to \mathbb{R}$ be a metric and define the distance between two sets $A, B \subseteq (M x ... x M)$ by

$$\text{dist}_\rho(A, B) := \min_{a \in A, b \in B} \rho(a, b).$$

Furthermore, define the Hausdorff distance between the two sets by $HD_\rho(A, B) := \max (\sup_{\alpha \in A} \text{dist}_\rho (\{\alpha\}, B), \sup_{b \in B} \text{dist}_\rho (\{b\}, A))$.

*Definition 3.* Given a finite sequence of discrete random variables $(X_1, ... , X_m)$, the maximal exchangeability distance is given by

$$ED^{max}_{X_1 \times ... \times X_m} = \frac{\sum_{\pi \in S_m} \sum_{\tau \in S_m} HD_\rho\left(\text{supp } X_{\pi(1)} \times ... \times X_{\pi(m)}, \text{supp } X_{\tau(1)} \times ... \times X_{\tau(m)}\right)}{\rho(1_m, M_m)|S_m|(|S_m| - 1)}$$

and the mean exchangeability distance is given by

$$ED^{mean}_{X_1 \times ... \times X_m}$$

$$= \frac{\sum_{\pi \in S_m} \sum_{\tau \in S_m} \mathbb{E}_{X_{\pi(1)} \times ... \times X_{\pi(m)}}\left[\text{dist}_\rho\left(X_{\pi(1)} \times ... \times X_{\pi(m)}, \text{supp } X_{\tau(1)} \times ... \times X_{\tau(m)}\right)\right]}{\rho(1_m, M_m)|S_m|(|S_m| - 1)}$$

Here, $1_m$=(1,...,1) and $M_m$=(M,...,M).

Clearly, $ED^{max}_{X1x...xXm} = ED^{max}_{X\Pi(1)x...xX\Pi(m)}$ for any $\pi \in S_m$, and $ED^{max}_{X1x...xXm}$ if and only if $(X_1, ... , X_m)$ is weakly exchangeable, and the same is true for $ED^{max}_{Xx...xXm}$.

For the purpose of this application, we will mainly consider exchangeability of pairs of discrete random variables with values in M = {1, ..., $M$}. In this special case, since $X_1$ x $X_2$ is the reflection of $X_2$ x $X_1$ with respect to the line {(x,y) $\in \mathbb{R}^2$; y = x}, we get

$$P^{Var}_{X_1 \times X_2} = \left|Pr_{X_1 \times X_2} - Pr_{X_2 \times X_1}\right|$$

$$ED^{max}_{X_1 \times X_2} = \frac{HD_\rho(\text{supp } X_1 \times X_2, \text{supp } X_2 \times X_1)}{\rho((1,1),(M,M))}$$

$$ED^{mean}_{X_1 \times X_2} = \frac{\mathbb{E}_{X_1 \times X_2}\left[\text{dist}_\rho(X_1 \times X_2, \text{supp } X_2 \times X_1)\right]}{\rho((1,1),(M,M))}$$

**The exchangeability plot**

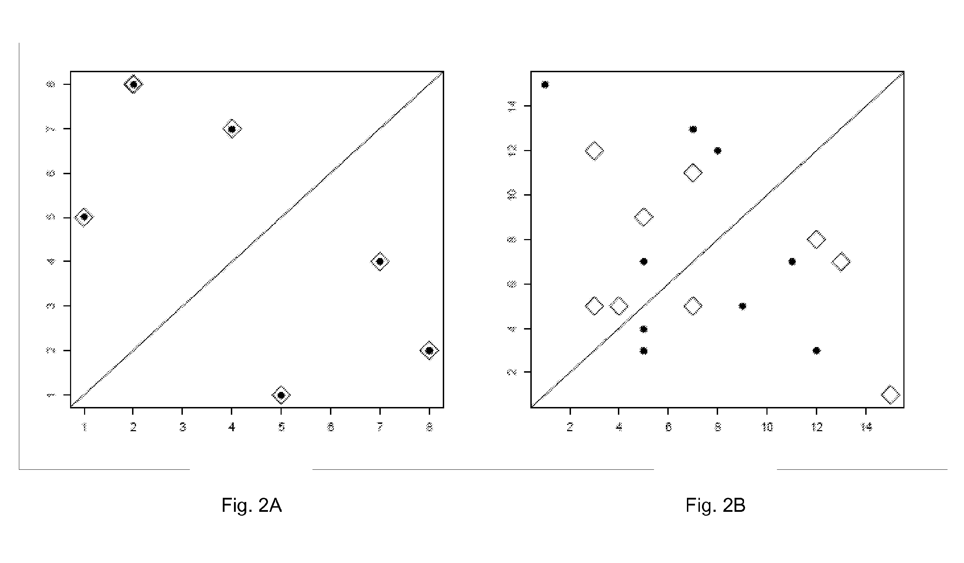

To illustrate the degree of weak exchangeability of a pair of discrete random variables visually we propose the exchangeability plot. The exchangeability plot for the random variables $X_1$ and $X_2$ is obtained by depicting both supp$X_1$ x $X_2$ and supp$X_2$ x $X_1$ in the same figure. A pair of random variables $(X_1,X_2)$ is weakly exchangeable if the two sets overlap completely. Fig. 2 shows exchangeability plots for two pairs of random variables. The pair in Fig. 2A is weakly exchangeable, while the pair in Fig. 2B is not.

**The exchangeability of genes**

In an embodiment, wherein the data is gene expression data we assume that we are given a universal set of M genes, denoted $\mathcal{G}$ = {$g_1$, ..., $g_M$}. Data in the form of a population (e.g. cancer patients and healthy control subjects) and a variable ranking method (e.g. a t-test contrasting the two groups in the population) is the starting point, called experiment, of the method. The sample space $\Omega$ consists of all possible rankings of the M genes, and the random variables $X_1, ... , X_M : \Omega \to \{1,...,M\}$ represent the ranking positions of the genes in $\mathcal{G}$. A finite set of genes $g_{i1}, ..., g_{im}$ is said to be (weakly) exchangeable if the corresponding sequence of random variables $(X_{i1}, ... , Xi_m)$ is (weakly) exchangeable. Intuitively, a set of genes is exchangeable if their positions in the variable ranking can be interchanged without changing the biological interpretation of the ranking.

Of course, we do not know the measures $Pr_{xi}$ for the variables in practice, so these have to be estimated. In this description we use subsampling to generate a collection of B data sets for which we compute gene rankings. From the B gene rankings, we construct a position vector $S_i$ for each gene $g_i$ by collecting all positions of the gene in the B rankings. The elements of a position vector $S_i$ are then samples of the random variable $X_i$. Combining two position

vectors $S_i$ and $S_j$ gives samples of the variables $X_i \times X_j$ and $X_j \times X_i$ which can be used to obtain estimates of $P^{Var}_{XixXj}$, $ED^{max}_{XixXj}$ and $ED^{mean}_{XixXj}$.

We now introduce another measure of exchangeability which is especially adapted to the study of ranked gene lists. The rationale behind this measure is that we may not want two genes to obtain a small exchangeability distance if they always appear in the same order in the rankings. For example, a gene which is always ranked first is not highly exchangeable with a gene that is always ranked second, since the first gene is clearly more strongly related to the response than the second. The new measure penalizes such situations in the computation of the exchangeability distance. For a pair of random variables $(X_1, X_2)$ we define a new set-valued random variable on $\Omega$ by

$$R(X_1 \times X_2)(\omega) := \left\{(x, y) \in \mathbb{M} \times \mathbb{M}; \operatorname{sign}(x - y) = \big(X_1(\omega) - X_2(\omega)\big)\right\}.$$

*Definition 4.* The one-sided mean exchangeability distance for a pair of discrete random variables $(X_1, X_2)$ is defined by

$$oED^{mean}_{X_1 \times X_2} := \frac{\mathbb{E}_{X_1 \times X_2}\left[\operatorname{dist}_\rho(X_1 \times X_2, \operatorname{supp} X_2 \times X_1 \cap R(X_1 \times X_2))\right]}{\rho\big((1,2),(M-1,M)\big)}$$

if $suppX_2xX_1 \cap R(X_1xX_2)(\omega) \neq \varnothing$ for all $\omega \in \Omega$, and $oED^{mean}_{X_1xX_2} = 1$ otherwise.

It is also possible to define a one-sided variant of the maximal exchangeability distance ($oED^{max}_{XixXj}$) in an analogous manner. We note that due to the normalization factors introduced in the estimates of weak exchangeability, all measures introduced above attain only values in [0, 1]. This allows us to define similarity measures (exchangeability scores) for pairs of genes as follows:

$$PS^{Var}_{X_i \times X_j} = 1 - P^{Var}_{X_i \times X_j}, \quad ES^{mean}_{X_i \times X_j} = 1 - ED^{mean}_{X_i \times X_j}, \quad ES^{max}_{X_i \times X_j} = 1 - ED^{max}_{X_i \times X_j},$$

$$oES^{mean}_{X_i \times X_j} = 1 - oED^{mean}_{X_i \times X_j}, \quad oES^{max}_{X_i \times X_j} = 1 - oED^{max}_{X_i \times X_j}.$$

Finally, we define normalized values of the exchangeability scores by comparing them to the corresponding values for pairs of random variables with some pre-specified distribution representing a null hypothesis of no association. In this paper, the main focus is on weak exchangeability of pairs of discrete random variables, in which case it is natural to compare to pairs of random variables $(Y_1, Y_2)$ uniformly distributed on a set $S \subseteq M \times M$ with cardinality equal to that of $suppX_1xX_2$. We show only the normalization for $oES^{mean}_{XixXj}$, the other scores can be normalized analogously.

*Definition 5.* The normalized one-sided mean exchangeability score for a pair of discrete random variables $(X_1,X_2)$ is defined by

$$noES^{mean}_{X_1 \times X_2} = \left(\frac{oES^{mean}_{X_1 \times X_2} - oES^{mean}_{Y_1 \times Y_2}}{1 - oES^{mean}_{Y_1 \times Y_2}}\right)_+$$

where $Y_1 \times Y_2$ is a uniformly distributed random variable on a set $S \subseteq M \times M$ where $|S| = |suppX_1 \times X_2|$, and $(a)_+ = \max(a,0)$.

We note that the measures of exchangeability depend on the number of genes in the ranking (M). For two genes having the exchangeability plot shown in Fig. 2A we get $noES^{mean}_{X_1xX_2} = 1.0$ irrespective of the number of genes since in this case, the distance between any value of $X_1 \times X_2$ and $suppX_2 \times X_1$ is zero. For the exchangeability plot in Fig. 2B we obtain $noES^{mean}_{X_1xX_2} = 0.17$ if M=15 and $noES^{mean}_{X_1xX_2} = 0.99$ if M=1,000.

**General idea**

In this section, we present a general framework for list representation and comparison, with a focus on an embodiment of the present invention applicable to analysis of biologic data, and in particular analysis of gene expression data. The lists are represented as vectors in $R^M$, where the entry in position $i$ gives the contribution of gene $g_i$. The vector representation allows us to compare both ranked and unranked gene lists within the same framework, using one of the many similarity or dissimilarity measures available to compare vectors in $R^M$. This is an advantage compared to existing methods for list comparison, which are specifically designed to compare certain types of lists. Our framework also provides a way to determine which genes are most important for explaining the similarity between two lists. Assume for example that some measure based on the scalar product in $R^M$ is used to measure the similarity between two vectors $x$ and $y$. Then the value of $x_i y_i$ is a measure of the influence of the $i$' th variable on the similarity between the two lists. Finally, ordering the genes by their weights in the vector provides a new ranking of the genes.

In an embodiment, as above, we have a universal set of $M$ genes, $\mathcal{G} = \{g_1, \dots, g_M\}$, where the genes are indexed in a fixed (but otherwise arbitrary) fashion. The universal set can be e.g. all genes on a microarray chip. An ordered (or unordered) gene list is then an ordered (or unordered) subset of the universal set.

Let $\ell \subseteq \mathcal{G}$ denote a list. If $\ell$ is ordered and if gene $g_i$ is contained in $\ell$, we denote its position by $\pi_\ell(i)$. If $g_i \notin \ell$, we define $\pi_\ell(i) = 0$. For an unordered list, we let $\pi_\ell(i) := \chi_\ell(g_i)$, where $\chi_\ell$ is the characteristic function of the set $\ell$. Given a list $\ell$ we define a corresponding list matrix $G_\ell$ as the product of three basic $M \times M$ matrices;

$$G_\ell := A_\ell V_\ell W_\ell$$

The three basic matrices are designed to represent different characteristics of $\ell$. We call $A_\ell$ the position matrix, $V_\ell$ the exchangeability matrix and $W_\ell$ the global weight matrix. From the list matrix we form a list vector $l_\ell := ((l_\ell)_1, \dots, (l_\ell)_M)$, by letting $(l_\ell)_i := h((G_\ell)_i)$ where $(G_\ell)_i$ is the $i$:th column of $G_\ell$ and h: $R^M \to R$ is a summarization function, e.g. a norm. The list vector will be used as the vector representation of the list. Once all lists of interest are represented by vectors in $\mathbb{R}^M$, we can define the similarity between them e.g. as the cosine of the angle between the corresponding list vectors, i.e.

$$s(\ell_1, \ell_2) = \frac{l_{\ell_1} \cdot l_{\ell_2}}{\|l_{\ell_1}\| \|l_{\ell_2}\|}$$

where $\cdot$ denotes the inner product in $\mathbb{R}^M$, and we can obtain a dissimilarity coefficient as

$$d(\ell_1, \ell_2) = 1 - s(\ell_1, \ell_2)$$

Choosing $A_\ell$, $V_\ell$, $W_\ell$, $h$ and the (dis)similarity coefficient on $\mathbb{R}^M$ suitably, most methods currently available for list comparison fit into this general framework.

**The position matrix**

The position matrix $A_\ell$ is defined as a diagonal matrix that contains information about the type of list (ordered or unordered) and the positions of the genes within the list. We define the diagonal elements (the position values) by $(A_\ell)_{ii} = u(r_i)$ where $r_i$ is the ranking statistic of gene $g_i$, and $u : \mathbb{R} \to \mathbb{R}$ is a monotone function.

For all genes not in the list, we define $(A_\ell)_{ii} = 0$. In this paper we mainly use rank-based functions, i.e. letting $(A_\ell)_{ii} = \tilde{u}(\pi_\ell(i))$ where $\tilde{u}: \mathbb{N} \to \mathbb{R}$ is a decreasing function of $\pi_\ell(i)$ if $\pi_\ell(i) > 0$, and $\tilde{u}(0) = 0$.

This means that the diagonal elements corresponding to genes in the top of the list $\ell$ are high, while the genes further down in the list obtain lower values. Furthermore, all genes not in the list have position value zero. We note that in some cases, other choices of position values may be better suited for unordered lists, where it may be desirable to give the genes different weights, e.g. depending on some external criterion, even though there is no specified ordering.

**The exchangeability matrix**

The exchangeability matrix $V_\ell$ carries information about the exchangeability between the genes in $\mathcal{G}$ in the specific experiment giving the list $\ell$. In an embodiment, we define the entry $(V_\ell)_{ij}$ to be the estimated normalized one-sided mean exchangeability score of $g_i$ and $g_j$ (i.e. $\widehat{noES}^{mean}_{X_i x X_j}$), so the diagonal elements are always 1. If $V_\ell$ is diagonal, i.e. $V_\ell = I_M$, then the only non-zero elements in the list vector are those corresponding to genes that are actually contained in $\ell$ and consequently only the genes that are present in the list affect its vector representation. However, if $V_\ell$ is not diagonal, there is a possibility that the vector representation of the list is extended, i.e. that it contains non-zero entries for genes which are not themselves present in the list, but are exchangeable with some gene in the list. The (absolute) weight of a gene in the list can also be increased if it is highly exchangeable with a gene with a higher (absolute) position value, since the high exchangeability indicates that the genes could as well have switched positions without changing the interpretation of the list.

We note that the general framework for list representation supports any matrix of gene similarities in the place of $V_\ell$. Thus, in an embodiment, $V_\ell$ could be defined from some kind of expert biological knowledge, e.g. concerning which genes are related to the same biological function. This could be used for example when comparing lists from different experiments to each other. In another embodiment, the positive part of the correlation matrix is used in place of $V_\ell$, since a high correlation between the expression levels of two genes is often considered to indicate similar biological functions of the genes.

**The global weight matrix**

The global weight matrix $W_\ell$ is a diagonal matrix that permits weighting the influence of the genes differently, depending on some informativeness or reliability estimate. For example, we may wish to downweight the influence of a gene that has a high probability to be present in an arbitrarily chosen list, since this gene is unspecific and may not give much relevant information about the similarity between a pair of lists.

The specific embodiment following description focuses on an embodiment of the present invention applicable to analysis of biologic data, and in particular analysis of gene expression data. However, it will be appreciated that the invention is not limited to this application but may be applied to many other data including for example meteorology and astronomy.

**Specific embodiment**

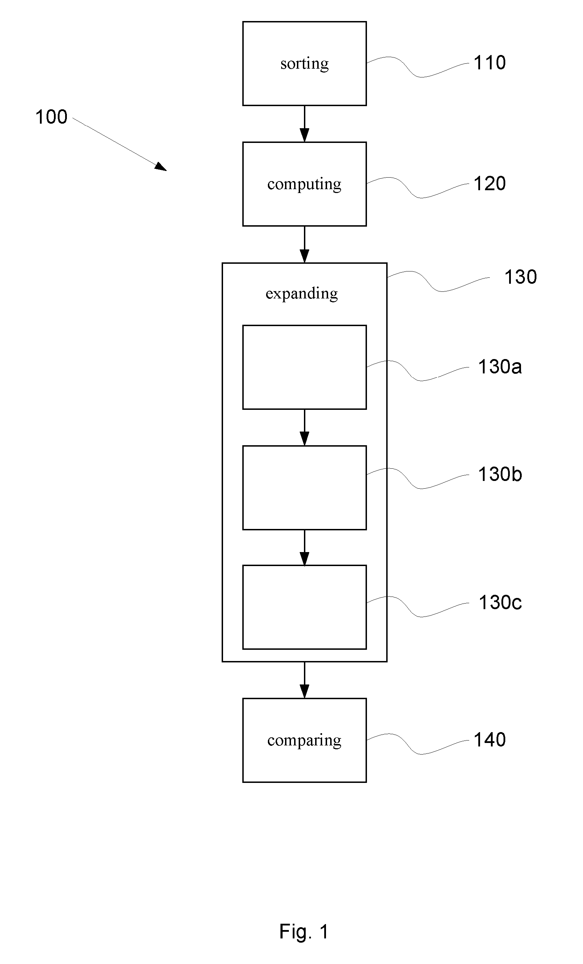

In an embodiment according to Fig. 1, data comprising a plurality of samples was obtained from a microarray study of lung adenocarcinoma patients, as provided in Bhattacharjee, A., Richards, W.G., Staunton, J., Li, C., Monti, S., Vasa, P., Ladd, C., Beheshti, J., Bueno, R., Gillette, M., Loda, M., Weber, G., Mark, E. J., Lander, E. S., Wong, W., Johnson, B. E., Golub, T. R., Sugarbaker, D. J., and Meyerson, M. (2001), Classification of human lung carcinomas by mRNA expression profiling reveals distinct adenocarcinoma subclasses. A ranking method, signal-to-noise ratio, was also provided.

The abovementioned data set consists of gene expression profiles from 31 patients with "good outcome" and 31 patients with "poor outcome". The data set was downloaded from http://www.broadinstitute.org/gsea/datasets.jsp (Lung_Boston_collapsed.gct), on January 11, 2011. In the downloaded file, original Affymetrix probe set IDs have been replaced with gene symbols and all probe sets matching to the same gene have been collapsed into one variable.

In the data set, 62 patient observations (31 with "good" outcome and 31 with "poor" outcome) thus gave 62 samples in form of observation vectors, comprising 5,217 measurement variables g, i.e. studied genes.

Signal-to-noise ratio (SNR) for each variable in the data was computed as

$$SNR = \frac{m_{poor} - m_{good}}{\sigma_{poor} + \sigma_{good}}$$

where $m_{poor}$ and $m_{good}$ is the mean value of the expression values in the "poor outcome" and "good outcome" group, respectively and $\sigma_{poor}$ and $\sigma_{good}$ is the standard deviation of the expression values in the "poor outcome" and "good outcome" group, respectively.

The variables were then sorted 110 by decreasing value of the computed statistic. In this way, each variable *i* obtains a position in the ranking. The variable with the highest (positive) SNR value obtained position 1, and the variable with the lowest (most negative) SNR value obtained position 5217. The experimental list is now the set of variables sorted in the indicated order, i.e. an ordered list $\ell$. The goal is to analyze the obtained list to enable robust biological conclusions.

Next, exchangeabilities between each pair of variables were computed 120 and collected in a 5217 x 5217 matrix. The exchangeabilities were computed in two steps.

Firstly, position vectors were computed by generating 20 slightly modified data sets by sub sampling the original data set, each time keeping 2/3 of the patients (randomly chosen) from each outcome group. For each modified data set, a signal-to-noise ratio (SNR) is computed for each variable as shown above, and used to rank the variables. The positions that a variable obtains in the 20 rankings are collected in a position vector for the variable. The position vector for variable *i* is denoted by $S_i$ ($1 \le i \le 5217$).

Secondly, for each pair of variables, the exchangeability score was estimated according to the following. For each pair of variables the joint position vectors were formed as:

$$S_{ixj} = \left\{ \left( (S_i)_k, (S_j)_k \right) \right\}_{k=1}^{20}$$

and

$$S_{jxi} = \left\{ \left( (S_j)_k, (S_i)_k \right) \right\}_{k=1}^{20}$$

These vectors describe the observed positions for the ordered pairs of variables. Then, the one-sided mean exchangeability distance between the two variables was estimated by

$$\dot{o}ED_{X_ixX_j}^{mean} = \frac{\sum_{k=1}^{20} \frac{1}{20} dist_\rho \left( \left\{ \left( (S_i)_k, (S_j)_k \right) \right\}, \left\{ \left( (S_j)_v, (S_i)_v \right) \right\}_{v=1}^{20} \cap R_{ij}(k) \right)}{\rho((1,2),(5216,5217))}$$

In this equation, $\rho$ is the standard Euclidean metric in $\mathbb{R}^2$ and, for two sets *A* and *B* in $\mathbb{R}^2$, $dist_\rho(A, B) = \min_{a \in A, b \in B} \rho(a, b)$ were defined.

Finally,

$R_{ij}(k) = \{(x, y) \in M \times M; sign(x - y) = sign((S_i)_k - (S_j)_k)\}$ denotes the points in $\{1,...5217\} \times \{1,...5217\}$ that are on the same side of the diagonal as the *k*'th point. In case $\{((S_j)_v,(S_i)_v)\}_{v=1}^B \cap R_{ij}(k) = 0$ for some *k*, the one-sided mean exchangeability distance between the two variables was defined to be 1.

From the one-sided mean exchangeability distance a one-sided exchangeability score was formed by

$$\dot{o}ES_{X_ixX_j}^{mean} = 1 - oED_{X_ixX_j}^{mean}.$$

The one-sided mean exchangeability score is normalized to give a normalized one-sided mean exchangeability score, by

$$\dot{n}oES_{X_ixX_j}^{mean} = \max\left( 0, \frac{\dot{o}ES_{X_ixX_j}^{mean} - \dot{o}ES_{Y_ixY_j}^{mean}}{1 - \dot{o}ES_{Y_ixY_j}^{mean}} \right).$$

The normalization constant $\dot{o}ES^{mean}{}_{Y_ixY_j}$ is the estimated value of the one-sided mean exchangeability score for variable pairs uniformly distributed on a set $S \subset \{1,...5217\} \times \{1,... 5217\}$ with 20 elements. The estimated value $\dot{n}oES^{mean}{}_{X_ixX_j}$ is then put in position (*i,j*) in the exchangeability matrix $V_\ell$.

Next, the ordered list $\ell$ of the data is expanded 130, thus combining the computed exchangeabilities with the information about the position of the variables in the experimental list to obtain a representation of the experimental list as a vector in $\mathbb{R}$ 5217. In an embodiment, this is done in several steps.

First, a diagonal position matrix $A_\ell$ is computed 130a, where the entry in the *i*'th row and column is derived from the position of the *i*'th variable in the list of experimental data. For all variables with a positive SNR as computed above (i.e. the variables in the top of the list),

$$A_{ii} = \frac{350}{(\pi(i)-1)^2+350}$$

is defined where $\pi(i)$ is the position of variable *i* in the list. For variables with a negative SNR,

$$A_{ii} = -\frac{350}{(5217 - \pi(i))^2 + 350}$$

is defined.

Second, a global weight matrix $W_\ell$ is computed 130b, which permits inclusion of a weight for each variable, which can be used to put more emphasis on e.g. genes that are known to be reliably measured. Since there is no such information in this example, the global weight matrix is taken to be the identity matrix.

Third, an expanded experimental list vector $l_\ell$ is computed 130c. The expanded list vector $l_\ell$ is taken as the vector representation of the experimental list in $\mathbb{R}$ 5217, according to the formula:

$$l_\ell = ((l_\ell)_1, \ldots, (l_\ell)_M)$$

by letting

$$(l_\ell)_i = h((G_\ell)_i)$$

where $G_\ell = A_\ell V_\ell W_\ell$, and where $(G_\ell)_i$ is the *i*'th column of $G_\ell$ and h: $\mathbb{R}$ M $\to$ $\mathbb{R}$ is defined by letting the *i*'th element of the list vector be the entry with the largest magnitude from column *i* in the list matrix. The list vector is called expanded since the value in each position is constructed using information from all the variables in the data set.

Finally, the expanded experimental list vector $l_\ell$ is compared 140 to another list vector comprising a plurality i of measurement variables g, i.e. the gene sets.

To compare the experimental list vector $l_\ell$ to the gene sets from MSigDB, vector representations of the gene sets are constructed as well. Since the gene sets are not generated directly from an experiment as the experimental list, there is not enough information to construct the exchangeability matrix as for the experimental list. Moreover, the gene sets are not ranked, i.e. the elements in a gene set do not have a specific order. Each gene set is therefore represented by a vector with 5217 elements, where the elements corresponding to the genes in the gene set are set to 1 and the others to 0.

Now, both the experimental list and the gene sets are represented by vectors of length 5217. A dissimilarity score is computed between two vectors by taking 1 minus the cosine of the angle between them, which is easily computed directly from the vectors. The dissimilarity score lies in the interval [0,2], where 0 is obtained only for identical list vectors. The output from this step is a list of dissimilarity scores (one for each gene set). The gene sets with the smallest dissimilarity score are most closely related to the genes in the top of the list (i.e. to the genes over expressed in the group with poor outcome relative to the group with good outcome), while the gene sets with the largest dissimilarity score are most closely related to the genes in the bottom of the list (i.e. to the genes over expressed in the group with good outcome relative to the group with poor outcome). In this way, conclusions can be drawn concerning which gene sets (and thereby which cellular functions) are most closely related to the two groups of observations.

These conclusions may be important for different therapeutical and/or diagnostical reasons, or for research purposes.

**Reference Example 1**

We will now estimate the robustness of the biological conclusions drawn in the abovementioned embodiment as described above. The effect of the stabilization will be estimated by repeating the same analysis without introducing the exchangeabilities, and both methods will be contrasted with what is obtained from Gene Set Enrichment Analysis (GSEA) (Subramanian et al., 2005). The robustness is estimated in the following way:

First, we generate ten slightly modified data sets from the original data through bootstrapping, i.e. sampling 31 observations from each outcome group ("good" and "poor") with replacement. Hence, some patient may be included more than once in the same modified data set.

Second, we compute two list vectors for each bootstrapped data set. The first list vector, the expanded list vector, is computed by sorting 110, computing 120 an exchangeability score and expanding 130, as described above. The second list vector, the non-expanded list vector, is obtained in the same way but using the identity matrix instead of the exchangeability matrix $V_\ell$, In this way, each entry in the list vector is affected only by the position of the corresponding variable.

Third, we compute the distances between the two list vectors and each of the gene sets by comparing 140 them for each of the ten bootstrapped data sets. The ten gene sets that have the shortest distance to the respective list vector and the ten gene sets that have the longest distance to the respective list vector are stored. For each pair of bootstrapped data sets, we compute the overlap between the collections of gene sets with shortest distances as well as the overlap between the collections of gene sets with longest distances to each of the two types of experimental list vectors (expanded and non-expanded).

For each of the ten bootstrapped data sets, we also apply GSEA using the R package provided by the authors at http://www.broadinstitute.org/gsea/downloads.jsp. For each data set we find the gene sets most related to each of the outcome groups and then we compute the overlap of these collections of gene sets for each pair of bootstrapped data sets. The GSEA algorithm uses a weighted Kolmogorov-Smirnov statistic to define an enrichment score (ES) for each gene set. These are normalized to eliminate the possible effect of the size of the gene sets, yielding normalized enrichment scores (NES) for the gene sets (denoted GSEANES).

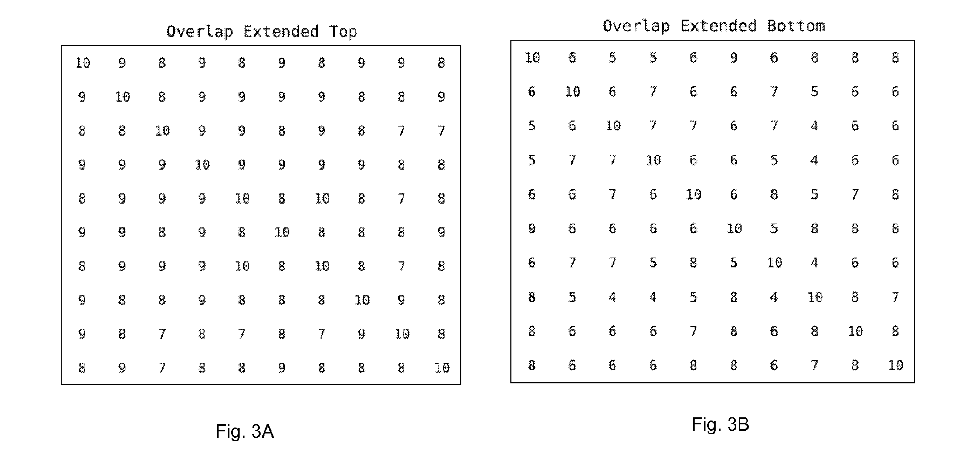

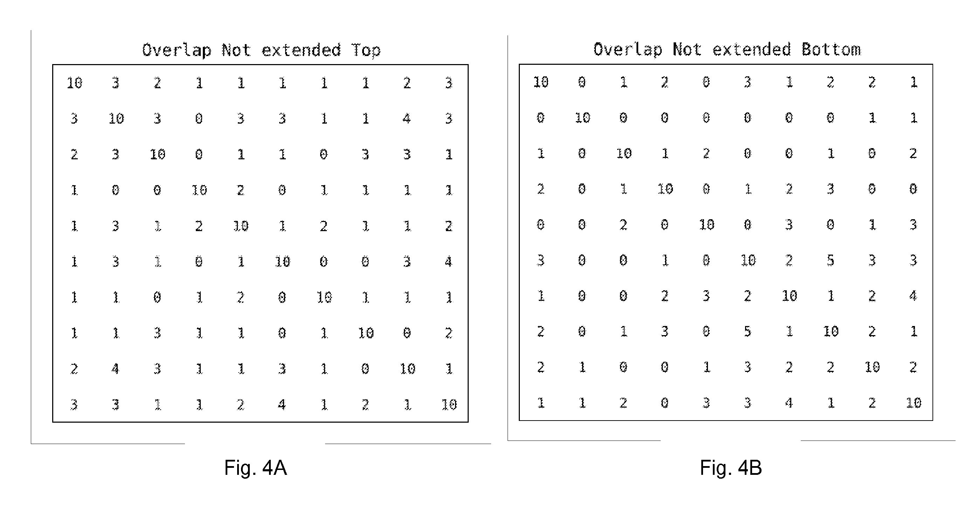

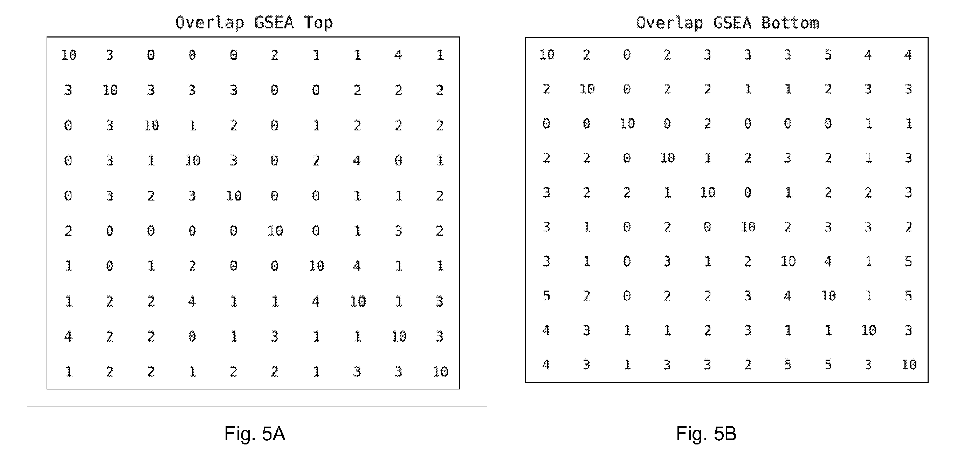

The result is seen in Figs 3-5. For each gene set collection (related to top (A), i.e. poor outcome, and bottom (B), i.e. good outcome, of the experimental list, respectively), the figures show the size of the overlap between the extracted gene sets for any two of the bootstrapped data sets. Fig. 3 is the result according to an embodiment of the invention, Fig. 4 is the result without expanding 130 the list and Fig. 5 is the result obtained using GSEA. The numbers represent degree of overlap (1-10, where 10 is maximum overlap). As can be seen, the robustness of the gene set rankings obtaining by comparing the expanded list vectors in this respect is remarkably high compared to that of the other two methods meaning that sampling variations have a much smaller impact on the resulting biological conclusions.

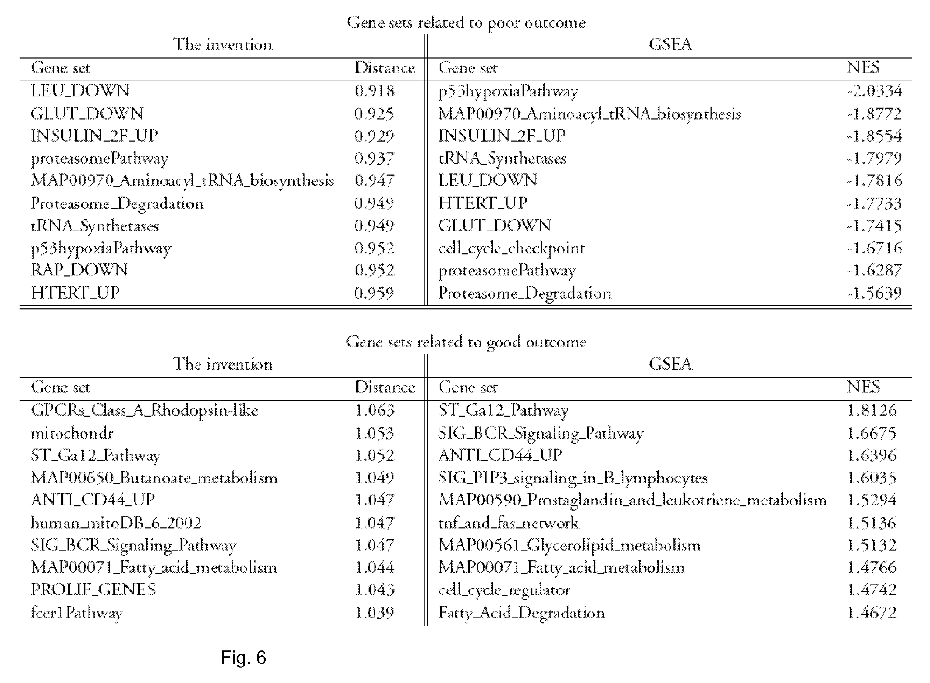

The gene sets are ranked by the distance to the extended list vector. By studying the gene sets with the shortest (or longest, respectively) distance to the experimental list we find the biological processes most related to the experimental contrast. Fig. 6 gives the closest and farthest gene sets with the method according to an embodiment of the invention "The invention" as well as the genesets with the largest positive and negative normalized enrichment score from GSEA, "GSEA". These are computed using the R implementation of the GSEA algorithm.

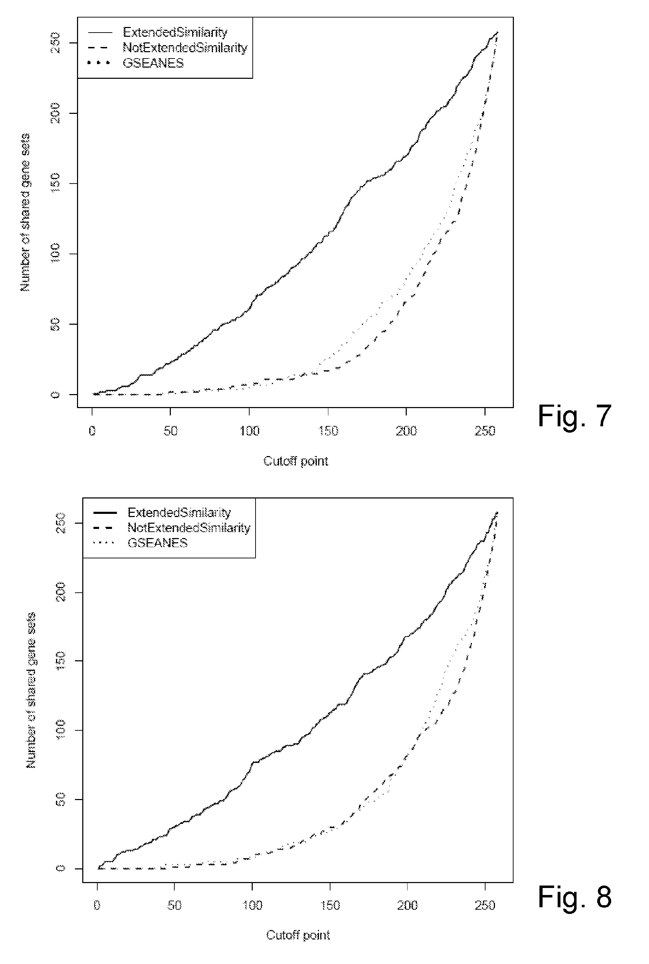

For all ranking metrics we also generate concordance plots for the gene set rankings obtained by ordering the gene sets by decreasing or increasing values of the respective ranking statistic, i.e. taking the direction of the association with the response into account. For each k the concordance plot shows the number of gene sets which are among the top-k ranked gene sets in all boot strapped data sets. The concordance plot where top gene sets are those positively associated with poor outcome is shown in Fig. 7, and the concordance plots for the rankings where the gene sets positively associated to good outcome are ranked in the top is shown in Fig. 8. In both plots, the number of shared gene sets is on the y axis and cutoff point is on the x axis.

In both Fig. 7 and 8, it is seen that the gene set rankings obtaining by comparing the expanded list vectors, represented by a line (ExtendedSimilarity) are more stable than the other two methods, GSEA represented by dotted line (GSEANES) and unexpanded list represented by a dashed line (NotExtendedSimilarity).

Thus, the ranking metrics based on the similarity to the extended list vector consistently provide reproducible rankings.

**Reference Example 2**

Even if stability is a tractable property of a gene list from an experiment, it is not the only one. We can obtain a perfectly robust gene list by always assigning each gene the same position, but the resulting list is useless from the point of view of biological interpretation. In this part we extract gene lists in three different ways and compare the biological

**EP 2 500 837 A1**

information contained in the top- and bottom-ranked genes in each list.

We compute three gene lists as follows. First, the non-expanded gene list which is the original list obtained by sorting the genes according to the SNR values. Then, the expanded gene list which is computed from the expanded list vector (obtained as described above) by sorting the genes according to their value in the expanded list vector. Finally, we compute an aggregated gene list by computing the median position in the position vector across 100 rankings from subsampled data sets for each gene and sorting the genes according to this, with the gene with lowest mean position in the top.

For each of the three gene lists, we extract a subset of the original data set consisting only of the top-k and bottom-k genes (for different choices of k). This small data set is fed into a centroid classifier (Schölkopf and Smola (2002): Learning with kernels, p. 4-5) and we estimate the classification ability of the subset by means of tenfold cross-validation. Table 1 shows the estimated classification accuracy for top and bottom genes from the expanded gene list, the non-expanded gene list and the aggregated gene list, as well as the mean classification accuracy for 20 random gene lists. The best performing subset for each k is highlighted in bold.

Apparently, the increased stability does not come at the price of reduced biological meaning since the top and bottom genes from the expanded gene list perform best in the classification task.

*Table 1.* The estimated classification accuracy for top and bottom genes of different lists.

|  | $k = 1$ | $k = 10$ | $k = 30$ | $k = 100$ |
|---|---|---|---|---|
| Extended | 0.344 | **0.774** | **0.837** | **0.832** |
| Non-extended | 0.344 | 0.583 | 0.606 | 0.566 |
| Aggregated | 0.410 | 0.565 | 0.617 | 0.566 |
| Random | **0.464** | 0.489 | 0.473 | 0.478 |

## Implementation

In an embodiment, a computer program product comprising computer program code means for executing the method according to some embodiments, when said computer program code means are run by an electronic device having computer capabilities.

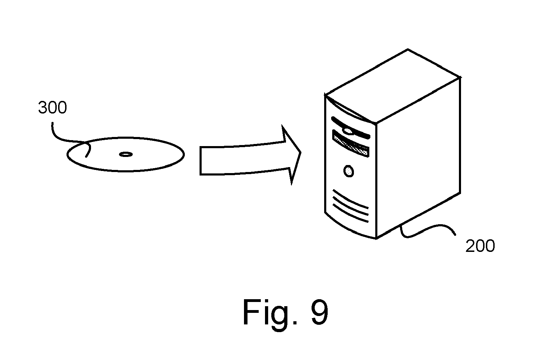

In an embodiment according to Fig. 9, a computer readable medium 300 is disclosed, having stored thereon a computer program product comprising computer program code means for executing the method according some embodiments, when said computer program code means are run by an electronic device 200 having computer capabilities.

Computer program, software program, program product, or software, in the present context mean any expression, in any programming language, code or notation, of a set of instructions intended to cause a system having computer processing capabilities to perform a particular function directly after conversion to another language, code or notation.

The electronic device 200 having computer capabilities may be any device normally used for performing the involved tasks, e.g. a hardware, such as a processor with a memory. The processor may be any of variety of processors, such as Intel or AMD processors, CPUs, microprocessors, Programmable Intelligent Computer (PIC) microcontrollers, Digital Signal Processors (DSP), etc. However, the scope of the invention is not limited to these specific processors. The memory may be any memory capable of storing information, such as Random Access Memories (RAM) such as, Double Density RAM (DDR, DDR2), Single Density RAM (SDRAM), Static RAM (SRAM), Dynamic RAM (DRAM), Video RAM (VRAM), etc. The memory may also be a FLASH memory such as a USB, Compact Flash, SmartMedia, MMC memory, MemoryStick, SD Card, MiniSD, MicroSD, xD Card, TransFlash, and MicroDrive memory etc. However, the scope of the invention is not limited to these specific memories.

The invention may be implemented in any suitable form including hardware, software, firmware or any combination of these. However, preferably, the invention is implemented as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors.

## Claims

**1.**  A computer-implemented method (100) for robust comparison of data comprising a plurality *i* of samples of each of a plurality of measurement variables g, based on a ranking method for sorting said data, said computer-implemented method comprising the steps
sorting (110) the data into a list $\ell$ according to the ranking provided by the ranking method;
computing (120) an exchangeability score of subsets of variables of the ordered data, resulting in an exchangeability matrix $V_\ell$;
expanding (130) the ordered lists $\ell$ of the data; and
comparing (140) the expanded experimental list vector $I_\ell$ with another list vector comprising a plurality of measurement variables g.

2. The computer-implemented method according to claim 1, wherein the expanding (130) comprises the steps computing (130a) a diagonal position matrix $A_\ell$, by defining diagonal elements as:

$$(A_\ell)_{ii} = u(r_i)$$

where $r_i$ is the ranking statistic of variable i used in sorting (110) the data into an ordered list $\ell$, and $u : \mathbb{R} \to \mathbb{R}$ is a monotone function;
computing (130b) a diagonal global weight matrix $W_\ell$, by weighting the data; computing (130c) an expanded experimental list vector $I_\ell$ according to a function:

$$f(A_\ell, V_\ell, W_\ell) = l_\ell.$$

3. The computer-implemented method according to claim 2, wherein computing (130c) an expanded experimental list vector $I_\ell$ is done according to the formula:

$$l_\ell = ((l_\ell)_1, \ldots, (l_\ell)_M)$$

by letting

$$(l_\ell)_i = h((G_\ell)_i)$$

where $G_\ell = A_\ell V_\ell W_\ell$; and $(G_\ell)_i$ is the $i$:th column of $G_\ell$ and $h$: $R^M \to R$.

4. The computer-implemented method according to claim 4, wherein the subsets of variables are subsets of variables with an exchangeability score exceeding a predefined threshold value.

5. The computer-implemented method according to any of the preceding claims, wherein the subsets of variables are pairs of variables.

6. The computer-implemented method according any of the preceding claims, wherein the ranking method is signal-to-noise ratio (SNR), fold change of average expression value, t-test, ANOVA, and non-parametric tests.

7. The computer-implemented method according to any of the preceding claims, wherein the computing (120) an exchangeability score of each pair of variables is total exchangeability variation score, PS, mean exchangeability score, ES(mean), maximal exchangeability score, ES(max), one-sided mean exchangeability score, oES(mean), one-sided maximal exchangeability score, oES(max), or normalized variants thereof.

8. The method according to any of the preceding claims, wherein the measurement variables are of the same kind.

9. The method according to any of the preceding claims, wherein the measurement variables are of different kinds.

10. The method according to claim 8, wherein the measurement variables are gene expression data.

**11.** A computer program product comprising computer program code means for executing the method according to any of the claims 1 to 10 when said computer program code means are run by an electronic device (200) having computer capabilities.

**12.** A computer readable medium (300) having stored thereon a computer program product comprising computer program code means for executing the method according to any of the claims 1 to 10 when said computer program code means are run by an electronic device (200) having computer capabilities.

**13.** A computer (200) configured to perform the method according to any of the claims 1 to 10.

**14.** A method for determining a relationship between a plurality of physical and/or biological parameters, comprising obtaining a plurality $i$ of samples of each of a plurality of measurement variables g, and a ranking method; and analyzing the samples of measurements using the method according to any of the claims 1 to 10.

**15.** A method according to claim 14, wherein the physical parameters are gene expression data.

100

110 — sorting

120 — computing

130 — expanding

130a

130b

130c

140 — comparing

Fig. 1

Fig. 2A

Fig. 2B

Overlap Extended Top

| 10 | 9 | 8 | 8 | 9 | 9 | 8 | 8 | 9 | 8 |
|----|---|---|---|---|---|---|---|---|----|
| 9 | 10 | 8 | 9 | 9 | 8 | 9 | 9 | 8 | 9 |
| 8 | 8 | 10 | 9 | 8 | 8 | 9 | 8 | 7 | 7 |
| 9 | 9 | 9 | 10 | 9 | 9 | 9 | 9 | 8 | 8 |
| 8 | 9 | 9 | 9 | 8 | 8 | 9 | 9 | 7 | 8 |
| 9 | 9 | 8 | 10 | 10 | 8 | 8 | 10 | 7 | 8 |
| 8 | 9 | 9 | 8 | 8 | 8 | 8 | 8 | 8 | 9 |
| 9 | 9 | 8 | 10 | 10 | 8 | 8 | 10 | 7 | 8 |
| 9 | 8 | 7 | 8 | 8 | 10 | 9 | 8 | 9 | 8 |
| 8 | 9 | 7 | 8 | 9 | 8 | 8 | 8 | 8 | 10 |

Fig. 3A

Overlap Extended Bottom

| 10 | 6 | 5 | 5 | 6 | 9 | 6 | 8 | 8 | 8 |
|----|---|---|---|---|---|---|---|---|----|
| 6 | 10 | 6 | 7 | 6 | 9 | 7 | 5 | 6 | 6 |
| 5 | 6 | 10 | 7 | 7 | 8 | 7 | 4 | 6 | 6 |
| 5 | 7 | 7 | 10 | 6 | 9 | 5 | 4 | 6 | 6 |
| 6 | 6 | 6 | 6 | 10 | 6 | 8 | 5 | 7 | 8 |
| 9 | 6 | 6 | 8 | 6 | 10 | 5 | 8 | 8 | 8 |
| 6 | 7 | 5 | 7 | 8 | 5 | 10 | 4 | 6 | 6 |
| 8 | 5 | 4 | 4 | 5 | 8 | 4 | 10 | 8 | 7 |
| 8 | 6 | 6 | 6 | 7 | 8 | 6 | 8 | 10 | 8 |
| 8 | 6 | 6 | 6 | 8 | 8 | 6 | 7 | 8 | 10 |

Fig. 3B

Overlap Not extended Top

| 10 | 3  | 2  | 1  | 1  | 1  | 1  | 1  | 2  | 3  |
|----|----|----|----|----|----|----|----|----|----|
| 3  | 10 | 3  | 0  | 3  | 3  | 1  | 1  | 4  | 3  |
| 2  | 3  | 10 | 0  | 1  | 1  | 0  | 3  | 3  | 1  |
| 1  | 0  | 0  | 10 | 2  | 0  | 1  | 1  | 1  | 1  |
| 1  | 3  | 1  | 2  | 10 | 1  | 1  | 1  | 1  | 2  |
| 1  | 3  | 1  | 0  | 1  | 10 | 0  | 0  | 3  | 4  |
| 1  | 1  | 0  | 1  | 2  | 0  | 10 | 1  | 1  | 1  |
| 1  | 1  | 3  | 2  | 1  | 1  | 1  | 10 | 0  | 1  |
| 2  | 4  | 3  | 1  | 3  | 1  | 0  | 0  | 10 | 2  |
| 3  | 3  | 1  | 1  | 4  | 1  | 2  | 1  | 1  | 10 |

Fig. 4A

Overlap Not extended Bottom

| 10 | 0  | 1  | 2  | 0  | 3  | 3  | 1  | 2  | 1  |
|----|----|----|----|----|----|----|----|----|----|
| 0  | 10 | 1  | 0  | 0  | 0  | 0  | 0  | 1  | 1  |
| 1  | 0  | 10 | 1  | 0  | 0  | 0  | 1  | 0  | 2  |
| 2  | 0  | 1  | 10 | 0  | 1  | 2  | 3  | 0  | 0  |
| 0  | 0  | 0  | 0  | 10 | 0  | 3  | 0  | 1  | 3  |
| 3  | 0  | 0  | 1  | 0  | 10 | 2  | 5  | 3  | 3  |
| 1  | 0  | 2  | 3  | 3  | 2  | 10 | 1  | 2  | 4  |
| 2  | 1  | 3  | 0  | 1  | 5  | 1  | 10 | 2  | 1  |
| 2  | 0  | 1  | 1  | 3  | 3  | 2  | 2  | 10 | 2  |
| 1  | 1  | 2  | 3  | 3  | 3  | 4  | 1  | 2  | 10 |

Fig. 4B

19

**Overlap GSEA Top**

| 10 | 3 | 0 | 0 | 0 | 2 | 1 | 1 | 4 | 1 |
|----|---|---|---|---|---|---|---|---|---|
| 3 | 10 | 3 | 3 | 3 | 0 | 0 | 2 | 2 | 2 |
| 0 | 3 | 10 | 1 | 2 | 0 | 1 | 2 | 2 | 2 |
| 0 | 3 | 1 | 10 | 3 | 0 | 2 | 4 | 0 | 1 |
| 0 | 3 | 2 | 3 | 10 | 0 | 0 | 1 | 1 | 2 |
| 2 | 0 | 0 | 0 | 0 | 10 | 0 | 1 | 3 | 2 |
| 1 | 0 | 1 | 2 | 0 | 0 | 10 | 4 | 1 | 1 |
| 1 | 2 | 2 | 4 | 1 | 1 | 4 | 10 | 1 | 3 |
| 4 | 2 | 2 | 0 | 1 | 3 | 1 | 1 | 10 | 3 |
| 1 | 2 | 2 | 1 | 2 | 2 | 1 | 3 | 3 | 10 |

Fig. 5A

**Overlap GSEA Bottom**

| 10 | 2 | 0 | 2 | 3 | 3 | 3 | 5 | 4 | 4 |
|----|---|---|---|---|---|---|---|---|---|
| 2 | 10 | 0 | 2 | 2 | 1 | 1 | 2 | 3 | 3 |
| 0 | 0 | 10 | 0 | 2 | 0 | 0 | 0 | 1 | 1 |
| 2 | 2 | 0 | 10 | 1 | 2 | 3 | 2 | 1 | 3 |
| 3 | 2 | 2 | 1 | 10 | 0 | 1 | 2 | 2 | 3 |
| 3 | 1 | 0 | 2 | 0 | 10 | 2 | 3 | 3 | 2 |
| 3 | 1 | 0 | 3 | 1 | 2 | 10 | 4 | 1 | 5 |
| 5 | 2 | 0 | 2 | 2 | 3 | 4 | 10 | 1 | 5 |
| 4 | 3 | 1 | 1 | 2 | 3 | 1 | 1 | 10 | 3 |
| 4 | 3 | 1 | 3 | 3 | 2 | 5 | 5 | 3 | 10 |

Fig. 5B

## Gene sets related to poor outcome

| The invention | | GSEA | |
|---|---|---|---|
| Gene set | Distance | Gene set | NES |
| LEU_DOWN | 0.918 | p53hypoxiaPathway | -2.0334 |
| GLUT_DOWN | 0.925 | MAP00970_Aminoacyl_tRNA_biosynthesis | -1.8772 |
| INSULIN_2F_UP | 0.929 | INSULIN_2F_UP | -1.8554 |
| proteasomePathway | 0.937 | tRNA_Synthetases | -1.7979 |
| MAP00970_Aminoacyl_tRNA_biosynthesis | 0.947 | LEU_DOWN | -1.7816 |
| Proteasome_Degradation | 0.949 | HTERT_UP | -1.7733 |
| tRNA_Synthetases | 0.949 | GLUT_DOWN | -1.7415 |
| p53hypoxiaPathway | 0.952 | cell_cycle_checkpoint | -1.6716 |
| RAP_DOWN | 0.952 | proteasomePathway | -1.6287 |
| HTERT_UP | 0.959 | Proteasome_Degradation | -1.5639 |

## Gene sets related to good outcome

| The invention | | GSEA | |
|---|---|---|---|
| Gene set | Distance | Gene set | NES |
| GPCRs_Class_A_Rhodopsin-like | 1.063 | ST_Ga12_Pathway | 1.8126 |
| mitochondr | 1.053 | SIG_BCR_Signaling_Pathway | 1.6675 |
| ST_Ga12_Pathway | 1.052 | ANTI_CD44_UP | 1.6396 |
| MAP00650_Butanoate_metabolism | 1.049 | SIG_PIP3_signaling_in_B_lymphocytes | 1.6035 |
| ANTI_CD44_UP | 1.047 | MAP00590_Prostaglandin_and_leukotriene_metabolism | 1.5294 |
| human_mitoDB_6_2002 | 1.047 | tnf_and_fas_network | 1.5136 |
| SIG_BCR_Signaling_Pathway | 1.047 | MAP00561_Glycerolipid_metabolism | 1.5132 |
| MAP00071_Fatty_acid_metabolism | 1.044 | MAP00071_Fatty_acid_metabolism | 1.4766 |
| PROLIF_GENES | 1.043 | cell_cycle_regulator | 1.4742 |
| fcer1Pathway | 1.039 | Fatty_Acid_Degradation | 1.4672 |

Fig. 6

EP 2 500 837 A1

Fig. 7

Fig. 8

300

200

Fig. 9

Europäisches
Patentamt

European
Patent Office

Office européen
des brevets

**EUROPEAN SEARCH REPORT**

Application Number

EP 11 15 7794

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/018967 A1 (BALINSKI MICHEL L [FR] ET AL) 15 January 2009 (2009-01-15) | 1-13 | INV. G06F19/24 |
| A | * abstract; claims 1-24; figures 1-3 * <br> * paragraphs [0032] - [0090] * <br>----- | 14,15 | ADD. G06F19/20 |
| X | WO 2005/017807 A2 (ICONIX PHARM INC [US]; EL GHAOUI LAURENT [US]; NATSOULIS GEORGES [US]) 24 February 2005 (2005-02-24) | 1-13 | |
| A | * the whole document * <br>----- | 14,15 | |
| T | SONESON C ET AL: "A FRAMEWORK FOR LIST REPRESENTATION, ENABLING LIST STABILIZATION THROUGH INCORPORATION OF GENE EXCHANGEABILITIES", <br>, <br>16 March 2011 (2011-03-16), pages 1-34, XP002651054, <br>Retrieved from the Internet: <br>URL:http://arxiv.org/PS_cache/arxiv/pdf/11 03/1103.2876v1.pdf <br>[retrieved on 2011-07-18] <br>----- | | |
| X | LICAMELE LOUIS ET AL: "Indirect two-sided relative ranking: a robust similarity measure for gene expression data", BMC BIOINFORMATICS, BIOMED CENTRAL, LONDON, GB, <br>vol. 11, no. 1, 17 March 2010 (2010-03-17) <br>, page 137, XP021071484, <br>ISSN: 1471-2105, DOI: <br>10.1186/1471-2105-11-137 <br>* the whole document * <br>----- | 1-15 | TECHNICAL FIELDS SEARCHED (IPC) <br><br>G06F <br>G06Q |

The present search report has been drawn up for all claims

| Place of search | Date of completion of the search | Examiner |
|---|---|---|
| The Hague | 30 September 2011 | Swarén, Peter |

Europäisches
Patentamt

European
Patent Office

Office européen
des brevets

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

```
see sheet B
```

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

1. claims: 1-13

    a computer-implemented method for robust comparison of data based on a ranking method for sorting said data, with a computer and computer program product therefor;
    ---

2. claims: 14, 15

    a method for determining a relationship between a plurality of physical and/or biological parameters.
    ---

**ANNEX TO THE EUROPEAN SEARCH REPORT
ON EUROPEAN PATENT APPLICATION NO.**                EP 11 15 7794

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2011

| Patent document cited in search report | | Publication date | Patent family member(s) | Publication date |
|---|---|---|---|---|
| US 2009018967 | A1 | 15-01-2009 | NONE | |
| WO 2005017807 | A2 | 24-02-2005 | NONE | |

EPO FORM P0459

For more details about this annex : see Official Journal of the European Patent Office, No. 12/82